# EUROPÄISCHE PATENTSCHRIFT

(11) **EP 0 841 949 B1**
(45) Veröffentlichungstag und Bekanntmachung des Hinweises auf die Patenterteilung: **26.04.2006**
(21) Anmeldenummer: 96921895.7
(22) Anmeldetag: 05.07.1996
(51) Int. Cl.: A61K 47/48

(54) **VERWENDUNG VON SACCHARID-KONJUGATEN**
USE OF SACCHARIDE CONJUGATES
UTILISATION DE CONJUGUES DE SACCHARIDE

(30) Priorität: 05.07.1995 DE 19524515
(43) Veröffentlichungstag der Anmeldung: 20.05.1998
(73) Patentinhaber: Deutsches Krebsforschungszentrum Stiftung des öffentlichen Rechts, 69120 Heidelberg (DE)
(72) Erfinder: WIESSLER, Manfred, 69126 Heidelberg (DE); BEYREUTHER, Konrad, 69190 Heidelberg (DE)
(74) Vertreter: Schüssler, Andrea
(86) Internationale Anmeldenummer: PCT/DE1996/001206
(87) Internationale Veröffentlichungsnummer: WO 1997/002046

(56) Entgegenhaltungen:
- EP-A- 0 369 182
- EP-A- 0 526 649
- DD-A- 122 386
- CHEM. PHARM. BULL. (1995), 43(6), 920-6 CODEN: CPBTAL;ISSN: 0009-2363, 1995, XP002030260 FUJITA, TOMOYUKI ET AL: "Inhibitory effect of perillosides A and C, and related monoterpene glucosides on aldose reductase and their structure-activity relationships"
- CANCER CHEMOTHER. PHARMACOL. (1995), 35(5), 364-70 CODEN: CCPHDZ;ISSN: 0344-5704, 1995, XP002030261 POHL, J. ET AL: "D-19575 - a sugar-linked isophosphoramide mustard derivative exploiting transmembrane glucose transport"
- PHYTOCHEMISTRY (1992), 31(9), 3265-7 CODEN: PYTCAS;ISSN: 0031-9422, 1992, XP002030262 FUJITA, TOMOYUKI ET AL: "Perilloside A, a monoterpene glucoside from Perilla frutescens"
- DATABASE WPI Section Ch, Week 9518 Derwent Publications Ltd., London, GB; Class B03, AN 95-133680 XP002030267 & JP 07 048 264 A (NIPPON TERPENE KAGAKU KK) , 21.Februar 1995
- DATABASE WPI Section Ch, Week 9419 Derwent Publications Ltd., London, GB; Class B03, AN 94-156560 XP002030268 & JP 06 100 453 A (NIPPON TERPENE KAGAKU KK) , 12.April 1994
- CHEMICAL ABSTRACTS, vol. 81, no. 5, 5.August 1974 Columbus, Ohio, US; abstract no. 25895, OVRUTSKII, V. M.: "2,3,4,6-Tetraacetyl-.alpha.-D-glucosyl esters of N-aryl-N',N'-bis(2-chloroethyl)diamidophos phoric acid" XP002030265 & ZH. OBSHCH. KHIM. (1974), 44(4), 896-9 CODEN: ZOKHA4, 1974,
- CHEMICAL ABSTRACTS, vol. 69, no. 19, 4.November 1968 Columbus, Ohio, US; abstract no. 77679, GUDKOVA, I. P. ET AL: "Glycosyl phosphites and glycosyl phosphonites" XP002030266 & ZH. OBSHCH. KHIM. (1968), 38(6), 1340-4 CODEN: ZOKHA4, 1968,
- JOURNAL OF CONTROLLED RELEASE, Bd. 19, Nr. 1 / 03, 1.März 1992, Seiten 109-120, XP000261546 FRIEND D R ET AL: "DRUG GLYCOSIDES IN ORAL COLON-SPECIFIC DRUG DELIVERY"
- NUCLEAR MEDICINE AND BIOLOGY, Bd. 13, Nr. 1, 1986, EXETER GB, Seiten 7-12, XP002030263 R. PAUL ET AL.: "SCINTIGRAPHY WITH [18F]2-FLUORO-2-DEOXY-D-GLUCOSE OF CANCER PATIENTS."
- CANCER CHEMOTHER. PHARMACOL. (1996), 38(4), 355-365 CODEN: CCPHDZ;ISSN: 0344-5704, 1996, XP002030264 STUEBEN, JOERG ET AL: "Pharmacokinetics and whole-body distribution of the new chemotherapeutic agent.beta.-D-glucosylisophosporamide mustard and its effects on the incorporation of [methyl-3H]-thymidine in various tissues of the rat"

## Beschreibung

Die vorliegende Erfindung betrifft die Verwendung eines Saccharid-Konjugats zur Herstellung eines Arzneimittels oder diagnostischen Mittels zur Behandlung und/oder Diagnose von Tumorerkrankungen.

Allgemein besteht bei vielen Wirkstoffen, insbesondere solchen für die Krebstherapie und solchen, die in bestimmte Organe und deren Zellen, z.B. Gehirn, Neurone, Glia-, Astroglia- und andere nicht-neuronale Zellen, gelangen sollen, das Problem, daß diese schlecht an ihren Wirkort transportiert werden und daher erhebliche Nebenwirkungen haben.

Der vorliegenden Erfindung liegt somit die Aufgabe zugrunde, ein Mittel bereitzustellen, mit dem Wirkstoffe verschiedenster Art gezielt an ihren Wirkort transportiert werden können, so daß ihre Nebenwirkungen reduziert werden können.

Erfindungsgemäß wird dies durch die Gegenstände in den Patentansprüchen erreicht.

Gegenstand der vorliegenden Erfindung ist die Verwendung eines Saccharid-Konjugates wie in Patentanspruch 1 definiert zur Herstellung eines Arzneimittels oder diagnostischen Mittels zur Behandlung und/oder Diagnose von Tumorerkrankungen.

Die vorliegende Erfindung beruht auf der Erkenntnis des Anmelders, daß β-D-Glucose-Isophosphoramid gute anti-Tumor-Eigenschaften bei äußerst geringen Nebenwirkungen im Vergleich zu Cyclophosphoramid und Isophosphoramid hat. Ferner hat er erkannt, daß β-D-Glucose-Isophosphoramid durch einen gerichteten Transport in die Tumorzellen gelangt. Für diesen Transport hat sich ein Glucosetransporter als wesentlich erwiesen.

Auf der Basis vorstehender Erkenntnisse wurden Konjugate entwickelt, in denen ein oder mehrere Wirkstoffe an ein Monosaccharid, Disaccharid oder Oligosaccharid gebunden sind. Als Monosaccharid sind insbesondere Glucose, ganz besonders D-Glucose, Galaktose, Mannose, Arabinose, Xylose, Fucose, Rhamnose, 2-Amino-2-deoxyglucose, 2-Fluor-2-deoxyglucose, N-Acetyl-2-amino-2-deoxyglucose, N-Acetyl-2-amino-2-deoxygalaktose, Digitoxose und 2-Amino-2-deoxygalactose zu nennen. Als Disaccharid eignen sich insbesondere Maltose, Laktose oder Gentobiose, entweder 1,4- oder 1,6- verknüpft. Als Oligosaccharid ist insbesondere ein lineares und verzweigtes, z.B. di-, tri-, insbesondere N,N'-Di-2-chforethyl-(3,6-di-O-(β-D-gfucopyranosyl)-β-D-glucopyranosyl)-phosphorsäureesterdiamid, und tetraantennäres Oligosaccharid zu erwähnen.

Als Wirkstoff kommen jegliche therapeutisch und/oder diagnostisch verwendbare Substanzen in Frage. Dies sind insbesondere:
Antioxidantien, z.B. Cystein, N-Acetylcystein, *α*-Tocopherol (Vitamin E), Probucol, *α*-Lipponsäure, Limonen (Perillasäure); Xanthine, Carotinoide und Nitrone, Radiosensitizer, z.B. Misonidazol, und Inhibitoren der DNA-Reparatur, z.B. O⁶-Benzyldeoxyguanosin.

Die Bindung zwischen dem Saccharid und dem oder den Wirkstoffen kann in üblicher Weise vorliegen. Günstig ist es, wenn zumindest eine Bindung über die 1-Position des Saccharids vorliegt. Dies hat den Vorteil hat, daß am Wirkort, d.h. in der Zelle, der über die 1-Position des Saccharids gebundene Wirkstoff enzymatisch abgespalten und freigesetzt wird.

In einer bevorzugten Ausführungsform des erfindungsgemäßen Konjugats ist das Saccharid zumindest mit einem Wirkstoff über einen üblichen Linker verbunden. Als Linker eignen sich besonders Diole, ganz besonders kurzkettige Diole von 1,2-Diol, z.B. Ethylenglykol, bis 1,6-Hexandiol.

Erfindungsgemäße Konjugate reichern sich in Zellen, Organen und Geweben an, die Glucosetransporter und/oder verwandte Transporter davon aufweisen. Die Konjugate reichern sich insbesondere in Leber, Niere, Herz, Thymus, Schilddrüse, Darm und Gehirn sowie in allen Arten von Tumoren an.

Desweiteren können erfindungsgemäße Konjugate, wenn sie entsprechende Wirkstoffe enthalten, erfolgreich zur Therapie bzw. Diagnose von Tumoren verwendet werden. Als Beispiele von therapeutisch verwendbaren Wirkstoffen sind Radiosensitizer, wie Misonidazol, zu nennen, die zu einer tumorspezifischen Verstärkung der einer Radio- und/oder photodynamischen Therapie führen. Desweiteren sind besonders Inhibitoren der DNA-Reparatur, wie O⁶-Benzyldeoxyguanosin, zu erwähnen. Diese Wirkstoffe tragen zur tumorspezifischen Verminderung der DNA-Reparaturkapazität und damit zur Verbesserung einer Therapie durch alkylierende Tumor-Chemotherapeutika bei. Zur Tumortherapie und Prävention von Tumorerkrankungen eignen sich auch Konjugate, die Antioxidantien enthalten. Durch diese Wirkstoffe wird, wie vorstehend angegeben, die Bildung freier Radikale unterbunden, welche bekanntermaßen zu einer Zellschädigung führen und eine Entartung von Zellen begünstigen. Konjugate mit Antioxidantien können daher auch zum Schutz gesunder Zellen bei Chemo- und Bestrahlungstherapien verwendet werden. Ferner weisen jüngste Ergebnisse des Anmelders darauf hin, daß erfindungsgemäße, Tumortherapeutika enthaltende Konjugate weit weniger einer Resistenz gegenüber den Tumortherapeutika unterliegen als diese alleine.

Desweiteren eignen sich erfindungsgemäße Konjugate, die Nukleinsäuren, z.B. Oligonukieotide, als Wirkstoffe enthalten, zur Diagnose und/oder Therapie von Gen-Defekten, insbesondere solchen, die organspezifisch sind.

Die Erfindung wird durch die folgenden Beispiele erläutert, wobei Beispiel 2 zwar keinen der in Anspruch 1 genannten Wirkstoffe bettrift*,* aber die Herstellungsweise und Wirkungsweise der erfindunggemäßen Konjugate beispielhaft erlautert.

### Beispiel 1:Herstellung eines erfindungsgemäßen Konjugats, β-D-Galactopyranosid-[4-(1-methylethenyl)-1-cyclohexen-1-yl]methyl

In einem ausgeheizten 500 ml Dreihalskolben wurden unter Lichtschutz 250 ml abs. Diethylether, 7.5 g Silbercarbonat, 25 g fein gepulvertes Molekularsieb (4Å) und 2,3 ml (0,15 mol) des Antioxidanz S(-)Perillaalkohol 15 Min. gerührt. Dann wurden 6 g (0,15 mol) Tetraacetyl-*α*-bromgalactose in 100 ml abs. Diethylether in kleinen Portionen während einer Stunde hinzugefügt. Die Reaktion wurde dünnschichtchromatographisch (Kieselgel PE/Essigester 4:1 v/v) verfolgt. Nach 10 Std. wurde das Reaktionsgemisch filtriert und die festen Rückstände mit Aceton gewaschen. Danach wurde mit einer 5% NaHCO₃-Lösung und Wasser gewaschen, mit Na₂SO₄ getrocknet, im Vakuum eingeengt und säulenchromatographisch (Kieselgel PE/Essigsester 6:1 v/v) gereinigt. Es wurden 3,05 g des Anomerengemisches als farbloses Öl erhalten. Eine HPLC-Chromätographie zeigte ein Produktverhältnis von β:*α* = 2,92. Die Abspaltung der Acetylschutzgruppen erfolgte in nahezu quantitativer Ausbeute durch Umsetzung mit Triethylamin 1 Std. bei Raumtemperatur.

Das erhaltene Produkt hat die Strukturformel:

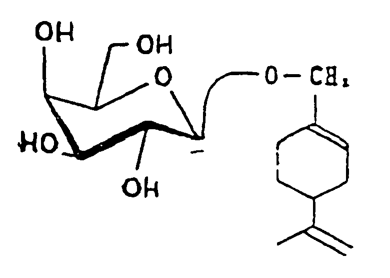

### Beispiel 2: Herstellung und Verwendung eines erfindungsgemäßen Konjugats, β-D-Glucosylisophosphoramid (β-D-Glc-IPM)

1,0 mM Glycosylimidat wurde in 20 ml Acetonitril gelöst. Nach Zugabe von 1,0 mM Isophosphoramid (Haloxan^{R}) wurde 6 Stunden unter Rückfluß in der Dunkelheit erhitzt. Nach Filtration und Einrotation wurde gemäß üblicher Verfahren an Kieselgel chromatographiert. Das erhaltene β-D-Glc-IPM hat die folgende Strukturformel:

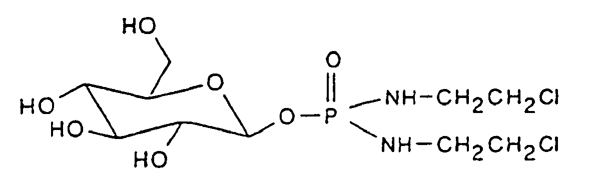

Dieses Konjugat wurde nach üblichen Verfahren mit ¹⁴C markiert und zur nachstehenden Behandlung von Ratten verwendet.

Als Ratten wurden weibliche Sprague-Dawley-Ratten (SD-Ratten; erhältlich von Charles River Wiga, Sulzfeld, Deutschland) und männliche Kopenhagen-Ratten (erhältlich von Harlan Sprague Dawley, Indianapolis, USA) eingesetzt.

Den Kopenhagen-Ratten wurden frische Stücke (2x2 mm) von Dunning-Prostata-Tumorgewebe transplantiert. Die Tumoren ließ man ca eine Woche wachsen bis diese gut tastbar, aber noch nicht nekrotisch waren.

Gruppen von 5 gesunden SD-Ratten erhielten i.v.-Injektionen von 315 mg/kg β-D-Glc-IPM, bzw. 56,2 mg/kg β-D-Glc IPM. 5 der tumor-tragenden Kopenhagen-Ratten erhielten 315 mg/kg β-D-Glc-IPM. Die radioaktive Dosis pro Injektion betrug etwa 20 *µ*Ci. Ein Tier jeder Gruppe wurde mit Co₂ 10 Minuten, 1 Std, 2 Std, 8 Std bzw. 24 Std. nach Verabreichung der Injektion getötet. Das Gewebe der Ratten wurde für eine übliche Mikrotom-Präparation vorbereitet und die Schnitte wurden wie von Ullberg, S., (1977), In: Alvefeldt, O. (ed.) Special issue on whole-body autoradiographie. Science Tools, Bromma, Sweden, p.2). beschrieben, einer Autoradiographie unterworfen.

Es zeigte sich, daß sich das erfindungsgemäße Konjugat bereits nach 10 Minuten in Organen, insbesondere Leber, Nieren, Thymus und Schilddrüse, angereichert hatte. Zentrales Nervensystem, einschließlich Gehirn, wies das Konjugat nach 8 Stunden auf. Desweiteren fand sich auch eine starke Anreicherung des Konjugats im Tumorgewebe.

## Patentansprüche

1. Verwendung eines Konjugats umfassend
- einen oder mehrere Wirkstoffe ausgewählt aus Antioxidantien, Radiosensitizern und Inhibitoren der DNA-Reparatur, und
- ein Monosaccharid, Disaccharid oder Oligosaccharid, wobei das Monosaccharid ausgewählt ist aus Glucose, Galaktose, Mannose, Arabinose, Xylose, Fucose, Rhamnose, 2-Amino-2-deoxyglucose, 2-Fluor-2-deoxyglucose, N-Acetyl-2-amino-2-deoxyglucose, N-Acetyl-2-amino-2-deoxygalaktose, 2-Amino-2-deoxygalaktose und Digitoxose
zur Herstellung eines Arzneimittels oder diagnostischen Mittels zur Behandlung und/oder Diagnose von Tumorerkrankungen.

2. Verwendung nach Anspruch 1, **dadurch gekennzeichnet, daß** mindestens ein Wirkstoff über die 1-Position des Saccharids gebunden ist.

3. Verwendung nach Anspruch 1, **dadurch gekennzeichnet, daß** das Disaccharid ausgewählt ist aus Maltose, Laktose und Gentobiose.

4. Verwendung nach Anspruch 1, **dadurch gekennzeichnet, daß** das Oligosaccharid ein verzweigtes Oligosaccharid, insbesondere N,N'-Di-2-chlor-ethyl(3,6-di-O-(*β*-D-glucopyranosyl)-*β*-D-glucopyranosyl)-phosphorsäureesterdiamid ist.

5. Verwendung nach Anspruch 1, **dadurch gekennzeichnet, daß** das Antioxidanz ausgewählt ist aus Cystein, N-Acetylcystein, *α*-Tocopherol (Vitamin E), Probobocol, *α*-Lipponsäure, Limonen (Perillasäure), Xanthinen, Carotinoiden und Nitronen.

6. Verwendung nach Anspruch 1, **dadurch gekennzeichnet, daß** der Radiosensitizer Misonidazol ist.

7. Verwendung nach Anspruch 1, **dadurch gekennzeichnet, daß** der Inhibitor der DNA-Reparatur O⁶-Benzyl-desoxyguanosin ist.

8. Verwendung nach einem der Ansprüche 1-7, **dadurch gekennzeichnet, daß** das Saccharid und mindestens ein Wirkstoff über einen Linker verbunden sind.

9. Verwendung nach Anspruch 8, **dadurch gekennzeichnet, daß** der Linker aus kurzkettigen Diolen von 1,2-Diol bis 1,6 Hexandiol ausgewählt ist.

## Claims

1. Use of a conjugate comprising
- one or more agents selected from antioxidants, radiosensitizers and inhibitors of DNA repair, and
- a monosaccharide, disaccharide or oligosaccharide, wherein the monosaccharide is selected from the group consisting of glucose, galactose, mannose, arabinose, xylose, fucose, rhamnose, 2-amino-2-deoxyglucose, 2-fluoro-2-deoxyglucose,N-acetyl-2-amino-2-deoxyglucose, N-acetyl-2-amino-2-deoxygalactose,2-amino-2-deoxygalactose and digitoxose
for the production of a pharmaceutical or diagnostics for the treatment and/or diagnosis of tumor diseases.

2. Use according to claim 1, **characterised in that** at least one agent is linked via the 1-position of the saccharide.

3. Use according to claim 1, **characterised in that** the disaccharide is selected from maltose, lactose and gentiobiose.

4. Use according to claim 1, **characterised in that** the oligosaccharide is a branched oligosaccharide, in particular N,N'-di-2-chloroethyl- (3,6-di-O- (β-D-glucopyranosyl)-β-D-glucopyranosyl)phosphoric ester diamide.

5. Use according to claim 1, **characterised in that** the antioxidant is selected from cysteine, N-acetylcysteine, alpha-tocopherol (vitamin E), probucol, alpha-lipoic acid, limonene (perillic acid), xanthines, carotinoids, and nitrones.

6. Use according to claim 1, **characterised in that** the radiosensitizer is misonidazole.

7. Use according to claim 1, **characterized in that** the inhibitor of the DNA repair is O⁶-benzyl deoxyguanosine.

8. Use according to any one of claims 1 to 7, **characterised in that** the saccharide and at least one agent are linked via a linker.

9. Use according to claim 8, **characterised in that** the linker is selected from short-chain diols from 1,2-diol to 1,6-hexanediol.

## Revendications

1. Utilisation d'un conjugué comprenant :
- un ou plusieurs agents choisis parmi les antioxydants, les sensibilisateurs radio et les inhibiteurs de réparation de l'ADN, et
- un monosaccharide, un disaccharide ou bien un oligosaccharide, où le monosaccharide est choisi parmi le glucose, le galactose, le mannose, l'arabinose, le xylose, le fucose, le rhamnose, le 2-amino-2-déoxyglucose, le 2-fluoro-2-déoxyglucose, le N-acétyl-2-amino-2-déoxyglucose, le N-acétyl-2-amino-2-déoxygalactose, le 2-amino-2-déoxygalactose et la digitoxose,
pour la préparation d'un médicament ou bien d'un moyen de diagnostique pour le traitement et/ou le diagnostique des maladies tumorales.

2. Utilisation selon la revendication 1, **caractérisée en ce qu'**au moins un agent est lié dans la position 1 du saccharide.

3. Utilisation selon la revendication 1, **caractérisée en ce que** le disaccharide est choisi parmi le maltose, le lactose et le gentobiose.

4. Utilisation selon la revendication 1, **caractérisée en ce que** l'oligosaccharide est un oligosaccharide ramifié, en particulier l'ester diamide de l'acide phosphorique N,N'-di-2-chloro-et-hyl(3,6-di-O-(β-D-glucopyranosyl).

5. Utilisation selon la revendication 1, **caractérisée en ce que** l'antioxydant est choisi parmi la cystéine, la N-acétylcystéine, l'α-tocophérol (vitamine E), le probobocol, l'acide α-lipoïque, les limonènes (acide perilla), les xanthines, les carotinoïdes et les nitrons.

6. Utilisation selon la revendication 1, **caractérisée en ce que** le sensibilisateur radio est le misonidazole.

7. Utilisation selon la revendication 1, **caractérisée en ce que** l'inhibiteur du réparateur ADN est le O⁶-benzyl-déoxyguanosine.

8. Utilisation selon la revendication 1 à 7, **caractérisée en ce que** le saccharide et au moins un agent est lié par l'intermédiaire d'une chaîne.

9. Utilisation selon la revendication 8, **caractérisée en ce que** la chaîne est choisie parmi les diols à chaîne courte du 1,2-diol au 1,6-hexandiol.
